# EUROPÄISCHE PATENTANMELDUNG

(11) **EP 4 417 110 A1**
(43) Veröffentlichungstag der Anmeldung: **21.08.2024**
(21) Anmeldenummer: 24157018.3
(22) Anmeldetag: 12.02.2024
(51) Int. Cl.: A61B 5/00

(54) **OPTISCHES MEDIZINISCHES UNTERSUCHUNGSGERÄT**

(30) Priorität: 16.02.2023 DE 102023103906
(71) Anmelder: Heine Optotechnik GmbH & Co KG, 82205 Gilching (DE)
(72) Erfinder: Raab, Roman, 82205 Gilching (DE); Michel, Felix, 82205 Gilching (DE)
(74) Vertreter: Flach Bauer & Partner Patentanwälte mbB

(57) **Zusammenfassung**

Ein Untersuchungsgerät (10) für optische medizinische Untersuchungen, insbesondere ein Dermatoskop, hat eine Optik (14) und eine Beleuchtungseinrichtung (16), wobei die Optik (14) eine optische Achse (A) hat und einen Untersuchungsbereich (U) definiert. Die Beleuchtungseinrichtung (16) weist wenigstens eine erste Lichtquelle (30) und wenigstens eine zweite Lichtquelle (32) auf, wobei die wenigstens eine erste Lichtquelle (30) und die wenigstens eine zweite Lichtquelle (32) derart angeordnet sind, dass sie den Untersuchungsbereich (U) gemeinsam ausleuchten, und wobei die wenigstens eine erste Lichtquelle (30) dazu ausgebildet ist, weißes Licht zu emittieren und die wenigstens eine zweite Lichtquelle (32) dazu ausgebildet ist, Licht zu emittieren, das blauer ist als das von der wenigstens einen ersten Lichtquelle (30) emittierte weiße Licht. Das von der wenigstens einen zweiten Lichtquelle (32) emittierte Licht hat eine Wellenlänge von 400 nm bis 550 nm und/oder die Beleuchtungseinrichtung (16) weist mehrere erste Lichtquellen (30) und mehrere zweite Lichtquellen (32) auf, wobei jeweils eine der ersten Lichtquellen (30) und eine der zweiten Lichtquellen (32) nebeneinander angeordnet sind und ein Paar bilden, und wobei von den Paaren wenigstens ein Paar ein erstes Paar (36) ist und wenigstens ein anderes Paar ein zweites Paar (38) ist, wobei die Beleuchtungseinrichtung (16) wenigstens einen Polarisationsfilter (34) aufweist, der das wenigstens eine zweite Paar (38) bedeckt.

## Beschreibung

Die Erfindung betrifft ein Untersuchungsgerät für optische medizinische Untersuchungen, insbesondere ein Dermatoskop.

Optische Untersuchungsgeräte, insbesondere Dermatoskope, sind bekannt und weisen üblicherweise eine Optik sowie eine Beleuchtungseinrichtung auf, mit der ein Untersuchungsbereich U beleuchtet werden kann.

Insbesondere bei der Untersuchung der Haut ist es notwendig, dass der Untersuchungsbereich mit Licht mit verschiedenen Farbtemperaturen und verschiedenen Polarisationen beleuchtet werden kann, da verschiedene Strukturen der Haut bei unterschiedlichen Beleuchtungsverhältnissen am besten sichtbar sind.

Gleichzeitig müssen Untersuchungsgeräte jedoch besonders kompakt sein, um eine gute Bedienbarkeit zu gewährleisten.

Es ist daher Aufgabe der Erfindung, ein Untersuchungsgerät für optische medizinische Untersuchungen bereitzustellen, dass Licht mit verschiedenen Farbtemperaturen bereitstellen kann und dabei besonders kompakt ist.

Die Aufgabe wird gelöst durch ein Untersuchungsgerät gemäß Anspruch 1. Das Untersuchungsgerät für optische medizinische Untersuchungen, insbesondere ein Dermatoskop, hat eine Optik und eine Beleuchtungseinrichtung. Die Optik hat eine optische Achse hat und definiert einen Untersuchungsbereich. Die Beleuchtungseinrichtung weist wenigstens eine erste Lichtquelle und wenigstens eine zweite Lichtquelle auf, wobei die wenigstens eine erste Lichtquelle und die wenigstens eine zweite Lichtquelle derart angeordnet sind, dass sie den Untersuchungsbereich gemeinsam ausleuchten. Die wenigstens eine erste Lichtquelle ist dazu ausgebildet, weißes Licht zu emittieren und die wenigstens eine zweite Lichtquelle ist dazu ausgebildet, Licht zu emittieren, das blauer ist als das von der wenigstens einen ersten Lichtquelle emittierte weiße Licht. Das von der wenigstens einen zweiten Lichtquelle emittierte Licht hat eine Wellenlänge von 400 nm bis 550 nm und/oder die Beleuchtungseinrichtung weist mehrere erste Lichtquellen und mehrere zweite Lichtquellen auf, wobei jeweils eine der ersten Lichtquellen und eine der zweiten Lichtquellen nebeneinander angeordnet sind und ein Paar bilden, wobei von den Paaren wenigstens ein Paar ein erstes Paar ist und wenigstens ein anderes Paar ein zweites Paar ist, und wobei die Beleuchtungseinrichtung wenigstens einen Polarisationsfilter aufweist, der das wenigstens eine zweite Paar bedeckt.

Die Erfinder haben erkannt, dass es zur Bereitstellung von Licht mit verschiedenen Farbtemperaturen nicht notwendig ist, multichromatische Lichtquellen, beispielsweise Mehrfarben-LED, zu verwenden, sondern dass die notwendigen ausreichenden Änderungen der Farbtemperatur bereits mittels zweier Lichtquellen erreicht werden kann, die Licht mit unterschiedlichen Spektren emittieren. Dadurch können die Lichtquellen deutlich kompakter ausgebildet sein, da beispielsweise einfarbige LED deutlich kleiner sind als Mehrfarben-LED.

Der Begriff "blauer" ist beispielsweise im Sinn von Farbe bzw. Farbwahrnehmung zu verstehen und nicht im Sinn einer niedrigeren Wellenlänge des emittierten Lichtes. "Blauer" kann insbesondere eine höhere Farbtemperatur bedeuten.

Die Begriffe "Mehrfarben" und "einfarbig" beziehen sich nicht auf die zu einem Zeitpunkt emittierte Wellenlänge, sondern auf die Möglichkeit der Lichtquelle, die Farbe bzw. Farbtemperatur des emittierten Lichtes zu verändern bzw. das Fehlen einer solchen Möglichkeit.

Unter "Ausleuchten" wird im Rahmen dieser Offenbarung insbesondere verstanden, dass der Lichtkegel der wenigstens einen ersten Lichtquelle und der Lichtkegel der wenigstens einen zweiten Lichtquelle im Untersuchungsbereich überlappen, insbesondere im überwiegenden Teil des Untersuchungsbereichs, bevorzugt im gesamten Untersuchungsbereich.

Die Lichtquellen sind beispielsweise LED.

Der Untersuchungsbereich kann ein Bereich um die optische Achse herum sein mit dem Durchmesser der Optik.

In einer Ausgestaltung der Erfindung hat das von der wenigstens einen ersten Lichtquelle emittierte weiße Licht eine Farbtemperatur zwischen 4000K und 6000K, insbesondere zwischen 4500K und 5500K, bevorzugt von 5000K oder eine Farbtemperatur zwischen 2500K und 4500K hat, insbesondere zwischen 3000K und 4000K hat, bevorzugt von 3500K hat; und/oder das von der wenigstens einen zweiten Lichtquelle emittierte blauere Licht hat eine Wellenlänge von 450 nm bis 500 nm, insbesondere von 475 nm, oder eine Farbtemperatur zwischen 5500K und 7500K, insbesondere zwischen 6000K und 7000K, bevorzugt von 6500K. Auf diese Weise wird die optimale Beleuchtung für verschiedene Untersuchungen bereitgestellt.

Der Aspekt, wonach das von der wenigstens einen zweiten Lichtquelle emittierte blauere Licht eine Farbtemperatur zwischen 5500K und 7500K, insbesondere zwischen 6000K und 7000K, bevorzugt von 6500K hat, löst die Aufgabe selbständig und ist insbesondere unabhängig von dem Aspekt, wonach das von der wenigstens einen zweiten Lichtquelle emittierte Licht eine Wellenlänge von 400 nm bis 550 nm hat.

Um eine zentrale Bedienung zu ermöglichen, kann das Untersuchungsgerät eine Steuereinheit aufweisen, die die wenigstens eine erste Lichtquelle und die wenigstens eine zweite Lichtquelle steuert.

In einer Ausführungsform ist das Untersuchungsgerät, insbesondere die Steuereinheit, derart eingerichtet, dass bei aktivierter Beleuchtungseinrichtung die wenigstens eine erste Lichtquelle eingeschaltet ist, wodurch eine Ausgangsfarbtemperatur bestimmt ist. An diese kann sich der Benutzer zum Beispiel gewöhnen.

Die wenigstens eine erste Lichtquelle ist zum Beispiel immer oder zumindest für den überwiegenden Teil der verschiedenen Beleuchtungsmodi eingeschaltet.

In einer Ausgestaltung ist das Untersuchungsgerät, insbesondere die Steuereinheit, derart eingerichtet, dass die Farbtemperatur des von der Beleuchtungseinrichtung emittierten Lichtes in einem Farbtemperaturbereich eingestellt werden kann, wobei die untere Grenze des Farbtemperaturbereichs zwischen 4000K und 6500K, insbesondere zwischen 4500K und 6000K, bevorzugt bei 5000K liegt, und/oder die obere Grenze des Farbtemperaturbereichs zwischen 10000K und 14000K, insbesondere zwischen 11000K und 13000K, bevorzugt bei 12000K liegt. In diesen Farbtemperaturbereichen sind optische Untersuchungen optimal möglich.

Um die Farbtemperatur auf einfache Weise zu verringern, kann das Untersuchungsgerät, insbesondere die Steuereinheit, derart eingerichtet sein, dass die wenigstens eine zweite Lichtquelle zusätzlich zur wenigstens einen ersten Lichtquelle eingeschaltet werden kann.

Insbesondere ist die wenigstens eine zweite Lichtquelle stets nur zusätzlich zur wenigstens einen ersten Lichtquelle eingeschaltet.

In einer Ausgestaltung ist das Untersuchungsgerät, insbesondere die Steuereinheit, derart eingerichtet, dass die Lichtstärke des von der wenigstens einen zweiten Lichtquelle emittierten Lichtes veränderlich ist, insbesondere in mehreren diskreten Stufen. Dadurch kann die Farbtemperatur einfach und im Falle von diskreten Stufen auch wiederholbar eingestellt werden.

Beispielsweise ist die Lichtstärke des von der wenigstens einen ersten Lichtquelle emittierten Lichtes ebenfalls veränderlich, insbesondere in mehreren diskreten Stufen. Dadurch kann die Helligkeit im Untersuchungsbereich einfach und im Falle von diskreten Stufen auch wiederholbar eingestellt werden.

Insbesondere hat das von den ersten Lichtquellen emittierte Licht immer eine höhere Lichtstärke als das der zweiten Lichtquellen.

In einer Ausführungsform sind die ersten Lichtquellen und die zweiten Lichtquellen um eine optische Achse der Optik herum angeordnet.

Insbesondere ist die gleiche Anzahl an ersten Lichtquellen wie an zweiten Lichtquellen vorhanden.

Die Anordnung um die optische Achse herum ist beispielsweise ringförmig.

Die LED sind beispielsweise in einem Winkel von 35° bis 55°, insbesondere von 45° von der optischen Achse weg geneigt.

Die Paare können den gleichen Abstand zueinander in Umfangsrichtung haben.

Der Polarisationsfilter der Beleuchtungseinrichtung ist insbesondere ein linearer Polarisationsfilter.

Beispielsweise sind mehrere erste und zweite Paare vorgesehen, wobei alle zweiten Paaren vom Polarisationsfilter der Beleuchtungseinrichtung bedeckt sind.

In Rahmen dieser Offenbarung bedeutet "bedeckt" insbesondere, dass der Polarisationsfilter in den Lichtkegel der Lichtquellen der ersten und der zweiten Lichtquelle des Paares angeordnet ist.

Der Polarisationsfilter der Beleuchtungseinrichtung ist insbesondere zwischen den ersten und zweiten Lichtquellen und dem Untersuchungsbereich angeordnet.

Zur Verstärkung des Kontrastes bestimmter zu untersuchender Strukturen kann der wenigstens eine Polarisationsfilter der Beleuchtungseinrichtung durchtretendes Licht beispielsweise in einer ersten Polarisationsrichtung linear polarisieren, und/oder die Optik weist einen Polarisationsfilter auf, der durchtretendes Licht in einer zweiten Polarisationsrichtung linear polarisiert, insbesondere wobei die zweite Polarisationsrichtung senkrecht zur ersten Polarisationsrichtung ist, sodass der Polarisationsfilter der Optik Licht mit einer Polarisation in der ersten Polarisationsrichtung sperrt.

Für eine besonders gleichmäßige Ausleuchtung können die ersten Paare und die zweiten Paare in Umfangsrichtung im Wechsel angeordnet sein.

In einer Ausgestaltung weist die Beleuchtungseinrichtung mehrere separate Polarisationsfilter auf, wobei je einer der Polarisationsfilter eines der zweiten Paare bedeckt.

Die separaten Polarisationsfilter polarisieren durchtretendes Licht alle in der gleichen ersten Polarisationsrichtung.

In einer Ausgestaltung ist das Untersuchungsgerät, insbesondere die Steuereinheit, derart eingerichtet, dass entweder die ersten Lichtquellen und die zweiten Lichtquellen des wenigstens einen ersten Paares oder die ersten Lichtquellen und die zweiten Lichtquellen des wenigstens einen zweiten Paares eingeschaltet sind, wenn die Beleuchtungseinrichtung aktiviert ist, wodurch entweder polarisiertes Licht oder unpolarisiertes Licht bereitgestellt wird.

Beispielsweise sind nie Lichtquellen eines ersten Paares und eines zweiten Paares gleichzeitig eingeschaltet.

Insbesondere sind immer die Lichtquellen aller ersten Paare oder aller zweiten Paare eingeschaltet, wenn die Beleuchtungseinrichtung aktiviert ist.

In einer Ausgestaltung der Erfindung ist das Untersuchungsgerät, insbesondere die Steuereinheit, derart eingerichtet, dass, wenn die Lichtquellen des wenigstens einen ersten Paares ausgeschaltet werden, um die Lichtquellen des wenigstens einen zweiten Paares zu aktivieren, die Lichtquellen des wenigstens einen zweiten Paares so eingeschaltet werden, dass die Lichtstärken des von den Lichtquellen des zweiten Paares emittierten Lichtes den Lichtstärken des Lichtes entsprechen, das zuletzt von der entsprechende Lichtquelle des wenigstens einen ersten Paares emittiert wurde, und/oder umgekehrt. Auf diese Weise wird sichergestellt, dass sich nur die Polarisation des Lichtes im Untersuchungsbereich ändert, sodass der Benutzer keine weiteren Einstellungen mehr vornehmen muss.

Beispielsweise ist die Optik eine vergrößernde Optik, weist einen bildgebenden Sensor und/oder weist eine Kamera auf, um die Untersuchung zu vereinfachen.

Weitere Merkmale und Vorteile der Erfindung ergeben sich aus der nachfolgenden Beschreibung sowie aus den beigefügten Zeichnungen, auf die Bezug genommen wird. In den Zeichnungen zeigen:
- Fig. 1: ein Untersuchungsgerät gemäß einer Ausführungsform der Erfindung in einer perspektivischen Ansicht,
- Fig. 2: eine schematische Schnittansicht durch einen Teil des Untersuchungsgeräts gemäß Figur 1, und
- Fig. 3: eine konzeptionelle Darstellung der Beleuchtungseinrichtung und der Optik in einer Unteransicht des Untersuchungsgeräts gemäß Figur 1.

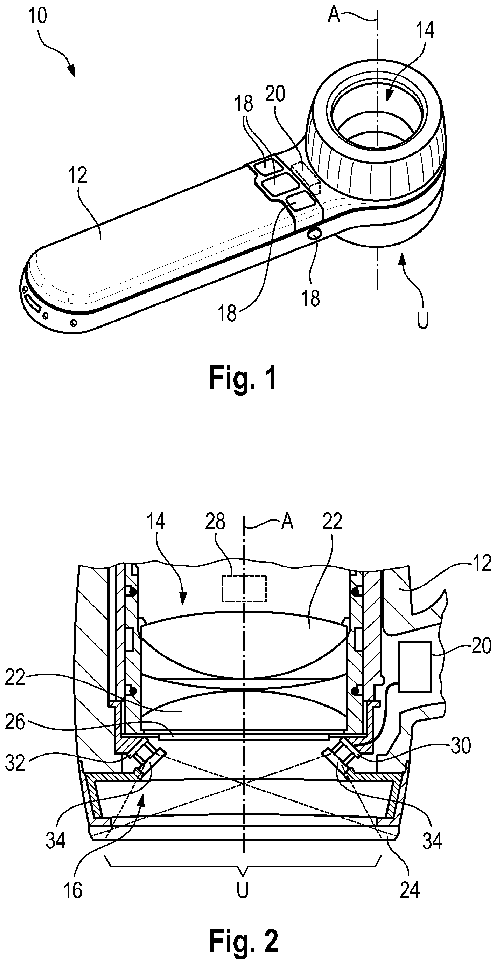

In Figur 1 ist ein Untersuchungsgerät 10 für optische medizinische Untersuchungen dargestellt.

Das Untersuchungsgerät 10 ist beispielsweise ein Dermatoskop und ist in der gezeigten Ausführungsform als Handgerät ausgeführt.

Das Untersuchungsgerät 10 weist ein Gehäuse 12, eine Optik 14, eine Beleuchtungseinrichtung 16 (Fig. 2), mehrere Taster 18 und eine Steuereinheit 20 auf.

Das Gehäuse 12 hat einen Griffbereich und einen Kopfbereich, wobei im Kopfbereich die Optik 14 und die Beleuchtungseinrichtung 16 angeordnet sind.

Am Griffbereich, insbesondere an dem dem Kopfbereich zugewandten Ende des Griffbereichs, sind die Taster 18 vorgesehen.

Die Taster 18 sind beispielsweise so angeordnet, dass sie mit dem Daumen bedient werden können, wenn der Griffbereich mittels der Hand gegriffen wird.

Die Optik 14, die Beleuchtungseinrichtung 16 und die Steuereinheit 20 sind innerhalb des Gehäuses 12 angeordnet. Zudem kann ein Energiespeicher, wie ein Akkumulator oder eine Batterie innerhalb des Gehäuses 12, insbesondere innerhalb des Griffbereiches vorgesehen sein.

In Figur 2 ist ein schematischer Schnitt durch den Kopfbereich des Gehäuses 12 sowie der darin befindlichen Optik 14 und der Beleuchtungseinrichtung 16 zu sehen.

Das Gehäuse 12 hat im Kopfteil einen Kanal, in dem die Optik 14 angeordnet ist.

Die Optik 14 umfasst mehrere Linsen 22, an der Patientenseite ein Deckglas 24, das den Kanal patientenseitig abschließt, und einen Polarisationsfilter 26.

Denkbar ist auch, dass die Optik 14 eine Kamera 28 hat (gestrichelt angedeutet). Auch ein bildgebender Sensor ist denkbar, beispielsweise anstelle der Kamera.

Die Optik 14 weist eine optische Achse A auf, die zentral durch die Linsen 22 verläuft.

Die Optik 14 bildet einen Bereich unmittelbar vor dem Deckglas 24 ab, der den Untersuchungsbereichs U darstellt.

Der Untersuchungsbereichs U erstreckt sich um die optische Achse A herum und hat in etwa den Durchmesser der Optik 14, d. h. der Linsen 22.

Die Linsen 22 sind derart ausgestaltet, dass für einen Benutzer auf der vom Untersuchungsbereich U abgewandten Seite das Bild des Untersuchungsbereichs U vergrößert dargestellt wird.

Der Polarisationsfilter 26 ist beispielsweise zwischen den Linsen 22 und dem Deckglas 24 angeordnet.

Im Kopfteil des Gehäuses 12 ist auch die Beleuchtungseinrichtung 16 vorgesehen.

Die Beleuchtungseinrichtung 16 erstreckt sich um die optische Achse A herum und ist zwischen dem Deckglas 24 und den Linsen 22 angeordnet.

Die Beleuchtungseinrichtung 16 weist mehrere erste Lichtquellen 30, mehrere zweite Lichtquellen 32 sowie mehrere Polarisationsfilter 34 auf.

Die Lichtquellen 30, 32 sind beispielsweise lichtemittierende Dioden (LED).

Die Lichtquellen 30, 32 sind um die optische Achse A herum angeordnet und schließen einen Winkel zwischen 35° und 55°, insbesondere 45° zur optischen Achse A ein.

Die Polarisationsfilter 34 sind vor den Lichtquellen 30, 32, d. h. zum Untersuchungsbereich U hin angeordnet.

Die Polarisationsfilter 34 sind im gezeigten Ausführungsbeispiel ebenfalls im gleichen Winkel wie die Lichtquellen 30, 32 zur optischen Achse A gewinkelt angeordnet.

Die Lichtquellen 30, 32 sind mit der Steuereinheit 20 elektrisch verbunden, wobei die Steuereinheit 20 dazu eingerichtet ist, die ersten Lichtquellen 30 und die zweite Lichtquellen 32 zu steuern, insbesondere getrennt voneinander bzw. in Gruppen, wie im Folgenden erläutert werden wird.

Die hierzu notwendige Energie stellt der Energiespeicher oder eine Kabelverbindung zu einer Energiequelle bereit.

Die ersten Lichtquellen 30 und die zweiten Lichtquellen 32 emittieren Licht in jeweils einem Lichtkegel (gestrichelt dargestellt), wobei die Lichtkegel der einzelnen Lichtquellen 30, 32 im Untersuchungsbereich U überlappen. Somit leuchten die ersten Lichtquellen 30 und die zweiten Lichtquellen 32 den Untersuchungsbereich U gemeinsam aus.

Insbesondere wird zumindest der überwiegende Teil des Untersuchungsbereichs U, bevorzugt jedoch der gesamte Untersuchungsbereich U durch die Lichtquellen 30, 32 gemeinsam ausgeleuchtet.

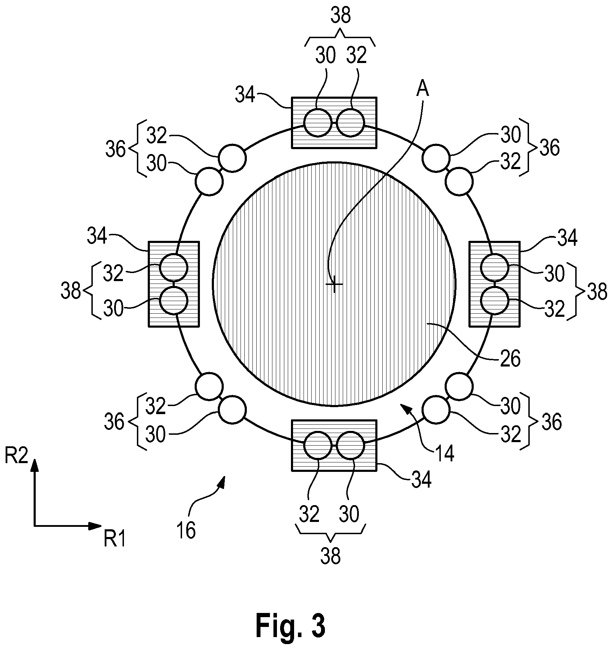

In Figur 3 ist eine Ansicht auf die Optik 14 und die Beleuchtungseinrichtung 16 von unten, d. h. durch das Deckglas 24 konzeptionell dargestellt.

Gut zu erkennen ist, dass die Optik 14, von der lediglich der Polarisationsfilter 26 zu sehen ist, kreisrund ausgeführt ist und sich die ersten Lichtquellen 30 und die zweiten Lichtquellen 32 entlang des Umfangs der Optik 14 erstrecken.

Die ersten Lichtquellen 30 und die zweiten Lichtquellen 32 sind in Umfangsrichtung um die optische Achse A angeordnet, im gezeigten Ausführungsbeispiel ringförmig.

Es ist die gleiche Anzahl an ersten Lichtquellen 30 wie an zweiten Lichtquellen 32 vorgesehen, im gezeigten Ausführungsbeispiel jeweils acht Stück.

Die ersten Lichtquellen 32 emittieren weißes Licht, zum Beispiel mit einer Farbtemperatur zwischen 4000 K und 6000 K, insbesondere zwischen 4500 K und 5500 K. Beispielsweise emittieren die ersten Lichtquellen 30 weißes Licht mit einer Farbtemperatur von 5000 K.

Die zweiten Lichtquellen 32 emittieren blaues Licht, beispielsweise mit einer Wellenlänge von 400 nm bis 550 nm, insbesondere von 450 nm bis 500 nm, bevorzugt von 475 nm.

Denkbar ist auch, dass die ersten Lichtquellen 32 Licht mit einer Farbtemperatur zwischen 2500 K und 4500 K, insbesondere zwischen 3000 K und 4000 K, beispielsweise von 3500 K emittieren, und die zweiten Lichtquellen 32 Licht mit einer Farbtemperatur zwischen 5500 K und 7500 K, insbesondere zwischen 6000 K und 7000 K, beispielsweise von 6500 K emittieren.

Die ersten Lichtquellen 30 und die zweiten Lichtquellen 32 sind in Umfangsrichtung um die optische Achse A im Wechsel angeordnet, wobei jeweils eine erste Lichtquelle 30 mit einer unmittelbar benachbarten zweiten Lichtquelle 32 ein Paar bildet.

Die Paare an Lichtquellen 30, 32 sind in Umfangsrichtung mit gleichem Abstand beabstandet. Beispielsweise ist der Abstand zwischen den Lichtquellen 30, 32 eines Paares geringer als der Abstand zwischen zwei benachbarten Paaren.

Die Polarisationsfilter 34 der Beleuchtungseinrichtung 16 sind für die Hälfte der Paare an Lichtquellen 30, 32 vorgesehen. Die Polarisationsfilter 34 sind voneinander separate Teile.

Im gezeigten Ausführungsbeispiel sind vier Polarisationsfilter 34 vorgesehen, wobei jeder dieser Polarisationsfilter 34 die Lichtquellen 30, 32 eines der Paare bedeckt.

Dies bedeutet, dass die Lichtkegel der Lichtquellen 30, 32 dieses Paares durch den entsprechenden Polarisationsfilter 34 verlaufen, sodass das von diesen Lichtquellen 30, 32 stammende Licht im Untersuchungsbereich U entsprechend polarisiert ist.

Durch die Polarisationsfilter 34 unterscheiden sich die Paare voneinander, wobei im Folgenden lediglich zur Unterscheidung die Paare von Lichtquellen 30, 32 ohne zugehörigen Polarisationsfilter als erste Paare 36 und die Paare von Lichtquellen 30, 32, die durch einen Polarisationsfilter 34 bedeckt sind, als zweite Paare 38 bezeichnet werden.

Die ersten Paare 36 und die zweiten Paare 38 sind in Umfangsrichtung um die optische Achse A im Wechsel angeordnet.

Denkbar ist, dass für die zweiten Paare 36 nur ein Polarisationsfilter 34 vorgesehen ist, der alle zweiten Paare 36 bedeckt.

Die Polarisationsfilter 34 weisen eine erste Polarisationsrichtung R1 auf, wobei das durch die Polarisationsfilter 34 hindurchtretende Licht linear in der ersten Polarisationsrichtung R1 polarisiert ist.

Die Polarisationsrichtung R1 ist in Figur 3 durch die Schraffur angedeutet.

Der Polarisationsfilter 26 der Optik 14 ist ebenfalls ein linearer Polarisationsfilter mit einer zweiten Polarisationsrichtung R2. Der Polarisationsfilter 26 polarisiert das ihn durchtretende Licht linear in Richtung der zweiten Polarisationsrichtung R2.

Die zweite Polarisationsrichtung R2 ist senkrecht zur ersten Polarisationsrichtung R1 und durch die Schraffur des Polarisationsfilters 26 angedeutet. Der Polarisationsfilter 26 der Optik 14 sperrt daher Licht mit einer Polarisation in der ersten Polarisationsrichtung R1, insbesondere Reflektionen der Lichtquellen 30, 32 der zweiten Paare 36. Daher können die Polarisationsfilter 34 der Beleuchtungseinrichtung 16 als Polarisator und der Polarisationsfilter 26 der Optik 14 als Analysator angesehen werden.

Während der Untersuchung mit dem Untersuchungsgerät 10 kann die Beleuchtungseinrichtung 16 aktiviert und deaktiviert werden, insbesondere mittels einer der Taster 18.

Die Steuerung der Lichtquellen 30, 32 erfolgt in Abhängigkeit der Betätigung der Taster 18 durch die Steuereinheit 20.

Mittels der Taster 18 können verschiedene Beleuchtungsmodi ausgewählt werden, in denen die ersten Lichtquellen 30 und die zweiten Lichtquellen 30 der verschiedenen Paare 36, 38 ein bzw. ausgeschaltet sind und Licht mit unterschiedlichen Lichtstärken emittieren.

Sobald die Beleuchtungseinrichtung 16 aktiviert ist, sind wenigstens die ersten Lichtquellen 30 der ersten Paare 36 oder die ersten Lichtquellen 30 der zweiten Paare 38 eingeschaltet.

In jedem der Beleuchtungsmodi sind entweder die ersten Lichtquellen 30 und gegebenenfalls die zweiten Lichtquellen 32 der ersten Paare 36 eingeschaltet und die Lichtquellen 30, 32 der zweiten Paare 38 ausgeschaltet, oder die Lichtquellen 30, 32 der ersten Paare 36 sind ausgeschaltet und die ersten Lichtquellen 30 und gegebenenfalls die zweiten Lichtquellen 32 der zweiten Paare 38 sind eingeschaltet.

Denkbar ist auch, dass die ersten Lichtquellen 30 der ersten Paare 36 oder die ersten Lichtquellen 30 der zweiten Paare 38 nicht immer, zumindest jedoch für mehr als die Hälfte der verschiedenen Beleuchtungsmodi eingeschaltet sind.

Die Lichtstärke des von den ersten Lichtquellen 30 emittierten Lichtes kann verändert werden. Beispielsweise können durch Betätigen einer der Taster 18 diskrete Stufen verschiedener Lichtstärken durchgeschaltet bzw. ausgewählt werden, um die Helligkeit im Untersuchungsbereich U zu verändern.

Insbesondere sind bei aktivierter Beleuchtungseinrichtung 16 stets erste Lichtquellen 30 eingeschaltet, entweder die ersten Lichtquellen 30 der ersten Paare 36 oder die der zweiten Paare 38.

Zu den eingeschalteten ersten Lichtquellen 30 der ersten bzw. zweiten Paare 36, 38 können zusätzlich die zweiten Lichtquellen 32 der entsprechenden Paare zugeschaltet werden. Dies kann ebenfalls durch Betätigen einer der Taster 18 geschehen.

Dabei ist die Lichtstärke des von den zweiten Lichtquellen 32 emittierten Lichtes veränderlich. Beispielsweise ist die Lichtstärke über mehrere diskrete Stufen veränderlich, die mittels einer der Taster 18 ausgewählt oder durchgeschaltet werden können.

Zum Beispiel hat das von den ersten Lichtquellen 30 emittierte Licht stets eine höhere Lichtstärke als das von den zweiten Lichtquellen 32 emittierte Licht.

Durch das Hinzuschalten der zweiten Lichtquellen 32 verändert sich die Farbtemperatur des Lichtes im Untersuchungsbereich U, da der Untersuchungsbereich U durch das Licht der ersten und der zweiten Lichtquellen 30, 32 ausgeleuchtet wird.

Durch Einschalten der zweiten Lichtquellen 32 wird die Farbtemperatur des Lichtes im Untersuchungsbereichs U ins Blaue verschoben, wodurch bestimmte Strukturen im zu untersuchenden Objekt, insbesondere der Haut, besser sichtbar werden.

Wenn die Lichtstärke des Lichtes der zweiten Lichtquellen 32 anschließend erhöht wird, wird die Farbtemperatur des Lichtes im Untersuchungsbereichs U weiter ins Blaue verschoben.

Beispielsweise liegt die untere Grenze des Farbtemperaturbereichs, der auf diese Weise erreicht wird bzw. von der Steuereinheit 20 zugelassen wird, zwischen 4000K und 6500K, insbesondere zwischen 4500K und 6000K, bevorzugt bei 5000K, und/oder die obere Grenze dieses Farbtemperaturbereichs liegt zwischen 10000K und 14000K, insbesondere zwischen 11000K und 13000K, bevorzugt bei 12000K.

Die Polarisation des Lichtes im Untersuchungsbereich U kann durch ein Umschalten zwischen den Paaren 36, 38 von Lichtquellen 30, 32 geschehen, also durch Ausschalten der Lichtquellen 30, 32 der einen Paare 36, 38 und Einschalten der Lichtquellen 30, 32 der anderen Paare 38, 36.

Das Umschalten zwischen den Lichtquellen 30, 32 der ersten Paare 36 bzw. zweiten Paare 38 kann durch Betätigen einer der Taster 18 ausgelöst werden.

Sind nur die Lichtquellen 30, 32 der ersten Paare 36 eingeschaltet, so ist das Licht im Untersuchungsbereich U unpolarisiert. Werden hingegen die Lichtquellen 30, 32 der zweiten Paare 38 verwendet, ist das Licht im Untersuchungsbereich U entlang der ersten Polarisationsrichtung R1 polarisiert.

Um den Wechsel von unpolarisiertem Licht zu polarisiertem Licht im Untersuchungsbereich U und umgekehrt für den Benutzer des Untersuchungsgeräts 10 so angenehm wie möglich zu gestalten, werden die ersten Lichtquellen 30 und zweiten Lichtquellen 32 der Paare 36, 38 die eingeschaltet werden so betrieben, wie die Lichtquellen 30, 32 der Paare 38, 36, die ausgeschaltet werden, bis dahin betrieben wurden, insbesondere in Bezug auf Lichtstärken.

Wird beispielsweise von unpolarisiertem Licht (d.h. den ersten Paaren 36) zu polarisiertem Licht (d.h. den zweiten Paaren 38) gewechselt, werden die Lichtstärken des Lichtes der ersten Lichtquellen 30 bzw. der zweiten Lichtquellen 32 der zweiten Paare 38 so eingestellt, dass sie den zuletzt verwendeten Lichtstärken des Lichtes der ersten Lichtquellen 30 bzw. der zweiten Lichtquellen 32 der ersten Paare 36 entsprechen.

Für das Umschalten von polarisiertem Licht zu unpolarisiertem Licht gilt entsprechend das Umgekehrte.

Mittels des Untersuchungsgeräts 10 kann somit die Farbtemperatur und auch die Polarisation des Lichtes im Untersuchungsbereich U eingestellt werden, ohne dass hierbei vielfarbige LEDs verwendet werden.

Auf diese Weise wird ein kompaktes und doch kostengünstiges Untersuchungsgerät 10 erhalten.

## Patentansprüche

1. Untersuchungsgerät für optische medizinische Untersuchungen, insbesondere Dermatoskop, mit einer Optik (14) und einer Beleuchtungseinrichtung (16),
wobei die Optik (14) eine optische Achse (A) hat und einen Untersuchungsbereich (U) definiert,
wobei die Beleuchtungseinrichtung (16) wenigstens eine erste Lichtquelle (30) und wenigstens eine zweite Lichtquelle (32) aufweist, wobei die wenigstens eine erste Lichtquelle (30) und die wenigstens eine zweite Lichtquelle (32) derart angeordnet sind, dass sie den Untersuchungsbereich (U) gemeinsam ausleuchten, und
wobei die wenigstens eine erste Lichtquelle (30) dazu ausgebildet ist, weißes Licht zu emittieren und die wenigstens eine zweite Lichtquelle (32) dazu ausgebildet ist, Licht zu emittieren, das blauer ist als das von der wenigstens einen ersten Lichtquelle (30) emittierte weiße Licht,
**dadurch gekennzeichnet,**
**dass** das von der wenigstens einen zweiten Lichtquelle (32) emittierte Licht eine Wellenlänge von 400 nm bis 550 nm hat und/oder
**dass** die Beleuchtungseinrichtung (16) mehrere erste Lichtquellen (30) und mehrere zweite Lichtquellen (32) aufweist, wobei jeweils eine der ersten Lichtquellen (30) und eine der zweiten Lichtquellen (32) nebeneinander angeordnet sind und ein Paar bilden, und wobei von den Paaren wenigstens ein Paar ein erstes Paar (36) ist und wenigstens ein anderes Paar ein zweites Paar (38) ist, wobei die Beleuchtungseinrichtung (16) wenigstens einen Polarisationsfilter (34) aufweist, der das wenigstens eine zweite Paar (38) bedeckt.

2. Untersuchungsgerät nach Anspruch 1, **dadurch gekennzeichnet, dass** das von der wenigstens einen ersten Lichtquelle (30) emittierte weiße Licht eine Farbtemperatur zwischen 4000K und 6000K hat, insbesondere zwischen 4500K und 5500K hat, bevorzugt von 5000K hat, oder eine Farbtemperatur zwischen 2500K und 4500K hat, insbesondere zwischen 3000K und 4000K hat, bevorzugt von 3500K hat; und/oder
dass das von der wenigstens einen zweiten Lichtquelle (32) emittierte Licht eine Wellenlänge von 450 nm bis 500 nm hat, insbesondere von 475 nm hat, oder eine Farbtemperatur zwischen 5500K und 7500K hat, insbesondere zwischen 6000K und 7000K hat, bevorzugt von 6500K hat.

3. Untersuchungsgerät nach Anspruch 1 oder 2, **dadurch gekennzeichnet, dass** das Untersuchungsgerät (10) eine Steuereinheit (20) aufweist, die die wenigstens eine erste Lichtquelle (30) und die wenigstens eine zweite Lichtquelle (32) steuert.

4. Untersuchungsgerät nach einem der vorhergehenden Ansprüche, **dadurch gekennzeichnet, dass** das Untersuchungsgerät (10), insbesondere die Steuereinheit (20), derart eingerichtet ist, dass bei aktivierter Beleuchtungseinrichtung (16) die wenigstens eine erste Lichtquelle (30) eingeschaltet ist.

5. Untersuchungsgerät nach einem der vorhergehenden Ansprüche, **dadurch gekennzeichnet, dass** das Untersuchungsgerät (10), insbesondere die Steuereinheit (20), derart eingerichtet ist, dass die Farbtemperatur des von der Beleuchtungseinrichtung (16) emittierten Lichtes in einem Farbtemperaturbereich eingestellt werden kann, wobei die untere Grenze des Farbtemperaturbereichs zwischen 4000K und 6500K, insbesondere zwischen 4500K und 6000K, bevorzugt bei 5000K liegt, und/oder die obere Grenze des Farbtemperaturbereichs zwischen 10000K und 14000K, insbesondere zwischen 11000K und 13000K, bevorzugt bei 12000K liegt.

6. Untersuchungsgerät nach einem der vorhergehenden Ansprüche, **dadurch gekennzeichnet, dass** das Untersuchungsgerät (10), insbesondere die Steuereinheit (20), derart eingerichtet ist, dass die wenigstens eine zweite Lichtquelle (32) zusätzlich zur wenigstens einen ersten Lichtquelle (30) eingeschaltet werden kann.

7. Untersuchungsgerät nach Anspruch 6, **dadurch gekennzeichnet, dass** das Untersuchungsgerät (10), insbesondere die Steuereinheit (20), derart eingerichtet ist, dass die Lichtstärke des von der wenigstens einen zweiten Lichtquelle (32) emittierten Lichtes veränderlich ist, insbesondere in mehreren diskreten Stufen.

8. Untersuchungsgerät nach einem der vorhergehenden Ansprüche, **dadurch gekennzeichnet, dass** die ersten Lichtquellen (30) und die zweiten Lichtquellen (32) um eine optische Achse (A) der Optik (14) herum angeordnet sind.

9. Untersuchungsgerät nach einem der vorhergehenden Ansprüche, **dadurch gekennzeichnet, dass** der wenigstens eine Polarisationsfilter (34) der Beleuchtungseinrichtung (16) durchtretendes Licht in einer ersten Polarisationsrichtung (R1) linear polarisiert, insbesondere wobei die Optik (14) einen Polarisationsfilter (26) aufweist, der durchtretendes Licht in einer zweiten Polarisationsrichtung (R2) linear polarisiert, bevorzugt wobei die zweite Polarisationsrichtung (R2) senkrecht zur ersten Polarisationsrichtung (R1) ist, sodass der Polarisationsfilter (26) der Optik (14) Licht mit einer Polarisation in der ersten Polarisationsrichtung (R1) sperrt.

10. Untersuchungsgerät nach einem der vorhergehenden Ansprüche, **dadurch gekennzeichnet, dass** die ersten Paare (36) und die zweiten Paare (38) in Umfangsrichtung im Wechsel angeordnet sind.

11. Untersuchungsgerät nach einem der vorhergehenden Ansprüche, **dadurch gekennzeichnet, dass** die Beleuchtungseinrichtung (16) mehrere separate Polarisationsfilter (34) aufweist, wobei je einer der Polarisationsfilter (34) der Beleuchtungseinrichtung (16) eines der zweiten Paare (38) bedeckt.

12. Untersuchungsgerät nach einem der vorhergehenden Ansprüche, **dadurch gekennzeichnet, dass** das Untersuchungsgerät (10), insbesondere die Steuereinheit (20), derart eingerichtet ist, dass entweder die ersten Lichtquellen (30) und die zweiten Lichtquellen (32) des wenigstens einen ersten Paares (36) oder die ersten Lichtquellen (30) und die zweiten Lichtquellen (32) des wenigstens einen zweiten Paares (38) eingeschaltet sind, wenn die Beleuchtungseinrichtung (16) aktiviert ist.

13. Untersuchungsgerät nach Anspruch 12, **dadurch gekennzeichnet, dass** das Untersuchungsgerät (10), insbesondere die Steuereinheit (20), derart eingerichtet ist, dass, wenn die Lichtquellen (30, 32) des wenigstens einen ersten Paares (36) ausgeschaltet werden, um die Lichtquellen (30, 32) des wenigstens einen zweiten Paares (38) zu aktivieren, die Lichtquellen (30, 32) des wenigstens einen zweiten Paares (38) so eingeschaltet werden, dass die Lichtstärken des von den Lichtquellen (30, 32) des zweiten Paares (38) emittierten Lichtes den Lichtstärken des Lichtes entsprechen, das zuletzt von der entsprechende Lichtquelle (30, 32) des wenigstens einen ersten Paares (36) emittiert wurde, und/oder umgekehrt.

14. Untersuchungsgerät nach einem der vorhergehenden Ansprüche, **dadurch gekennzeichnet, dass** die Optik (14) eine vergrößernde Optik ist, einen bildgebenden Sensor aufweist und/oder eine Kamera (28) aufweist.
